(19) Europäisches Patentamt
European Patent Office
Office européen des brevets

(11) EP 4 559 565 A1

(12) **EUROPEAN PATENT APPLICATION**

(43) Date of publication:
**28.05.2025 Bulletin 2025/22**

(21) Application number: **23212012.1**

(22) Date of filing: **24.11.2023**

(51) International Patent Classification (IPC):
***B01D 67/00*** *(2006.01)* ***B01D 39/20*** *(2006.01)*
***B01D 69/10*** *(2006.01)* ***B01D 71/40*** *(2006.01)*
***B01D 71/76*** *(2006.01)* ***G01N 33/49*** *(2006.01)*
***G01N 33/52*** *(2006.01)*

(52) Cooperative Patent Classification (CPC):
**B01D 69/106; B01D 39/2017; B01D 67/0088; B01D 69/108; B01D 71/40; B01D 71/401; B01D 71/76; G01N 33/491; G01N 33/525;**
B01D 2239/0421; B01D 2239/0492; B01D 2323/02; B01D 2323/30

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Roche Diagnostics GmbH**
  **68305 Mannheim (DE)**
  Designated Contracting States:
  **DE**
- **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC ME MK MT NL NO PL PT RO RS SE SI SK SM TR**
- **Albert-Ludwigs-Universität Freiburg**
  **79098 Freiburg (DE)**

(72) Inventors:
- **Lopez-Calle, Eloisa**
  **68305 Mannheim (DE)**
- **Marcinowski, Moritz**
  **68305 Mannheim (DE)**
- **Spinke, Jürgen**
  **68305 Mannheim (DE)**
- **Brandstetter, Thomas**
  **79110 Freiburg (DE)**
- **Frey, Holger**
  **79102 Freiburg (DE)**
- **Geid, Nicolas**
  **79106 Freiburg (DE)**
- **Rühe, Jürgen**
  **79356 Eichstetten (DE)**
- **Scherag, Frank**
  **79114 Freiburg (DE)**

(74) Representative: **Altmann Stößel Dick Patentanwälte PartG mbB**
**Theodor-Heuss-Anlage 2**
**68165 Mannheim (DE)**

(54) **METHOD OF COATING A GLASS FIBER FLEECE, COATED GLASS FIBER FILTER AND USES THEREOF**

(57) The present invention relates to a method for coating a glass fiber fleece comprising contacting the surface of a glass fiber fleece with a hydrophilic copolymer; and crosslinking the copolymer. The invention further relates to a glass fiber filter, comprising a glass fiber fleece, wherein the surface of the glass fiber fleece is coated with a hydrophilic copolymer; and to a device for biomedical filter applications comprising the same.

EP 4 559 565 A1

## Description

The present invention relates to a method for coating a glass fiber fleece comprising contacting the surface of a glass fiber fleece with a hydrophilic copolymer; and crosslinking the copolymer. The invention further relates to a glass fiber filter, comprising a glass fiber fleece, wherein the surface of the glass fiber fleece is coated with a hydrophilic copolymer; and to a device for biomedical filter applications comprising the same.

In biomedical analyses, in particular for diagnostic applications involving the analysis of liquid samples of bodily fluids such as blood, saliva, urine and the like, removing of cellular components from the sample prior to the analysis is often necessary. Filters comprising glass fiber fleece and filter membranes are commonly used for such purposes. In particular for blood diagnostics, separating blood plasma and/or serum from whole blood preparations, which are then subjected to further biomedical analyses, is required and usually involves filtering.

There are certain obstacles to address when choosing appropriate filter materials and filter methods: In addition to ensuring essentially complete separating of blood cells including erythrocytes from whole blood, the filtering process or material should not cause hemolysis, i.e., rupture of the blood cells during separation; otherwise, cellular fragments could interfere with accurate downstream measurements. Further, denaturing and/or unspecific binding of the components of the blood plasma or serum, such as proteins and lipids, should be avoided during the separation process. For rapid diagnostic applications, as in Point of Care (POC) applications, minimizing the time and the space required for the separation is also desirable. In a (POC) environment, usually, small test elements, or test strips, are used containing dried reagents capable of performing an analytical assay, in particular an immunoassay, when redissolved by a liquid sample. The test elements are typically based on an optical detectable reaction and evaluated visually or by optical instrumentation. Suitable filter elements are required to remove erythrocytes or other cellular or colored components from the liquid sample which would interfere with the optical readout of the test element.

At present, filters comprising glass fiber fleece offer large filtration volumes per time due to their three-dimensional glass fiber network and may therefore be faster, may require less space and are hence considered advantageous over conventional filter membranes for separating blood cells from whole blood preparations. However, glass fiber material and in particular material with a large surface area is prone to provide a large unspecific and undesired binding potential for sample components, for example for proteins. In particular, for subsequent biomedical or diagnostic analyses unspecific binding of potential analytes to the filter surface may falsify the analysis result and hence be detrimental. Therefore, specifically protein-repellent coatings for filter materials are desired. At present, chemical modification of the surface of filter materials is used in the art to reduce or avoid unwanted binding of blood components and/or analytes such as proteins. In particular, applying hydrophilic layers or coatings to filter materials including glass fiber material is proposed in the art to obtain protein-repellent filter materials suitable for biomedical applications such as blood filtration.

EP0457183 A1 suggests using a glass fiber comprising layer with an erythrocyte aggregating compound, wherein the glass fibers are coated with polyvinyl alcohol (PVA) or polyvinyl alcohol/polyvinyl acetate (PVAc) for separating red blood cells from plasma in whole blood preparations. The erythrocyte aggregating compound is applied to the coated glass fibers so that an additional erythrocyte aggregating layer is formed on the glass fibers. The application of more than one coating to the glass fibers requires a laborious multistep process. PVA layer typically adsorbs to the glass fiber surfaces, such that no permanent bonding to the surface of the glass fiber is established. Therefore, PVA or PVA/PVAc coatings typically wear off over time and may be prone to leaching.

EP1618940 A1 discloses a polymer coated glass fiber to obtain a glass fiber filter for blood filtration. Preferred polymers for surface coating of the glass fibers are biocompatible polymers including acrylate polymers such as poly (alkoxy acrylate). The coating is applied by immersing the acid cleaned glass fiber filter in a polymer solution. The glass fiber filter may be prepared by laminating a plurality of sheets that may be approximately 0.2 to 3 mm thick.

In JP2002350428 A, the applicants disclose a fiber containing filter layer comprising glass fiber coated with butoxy group-attached propanol and/or acrylamide such as N-(butoxymethyl)acrylamide to suppress hemolysis when separating plasma or serum from whole blood.

Coated glass fiber filter paper may be produced by immersion in an aqueous solution of N-(butoxymethyl) acrylamide followed by drying at a temperature of 70°C.

However, efficient and complete coating of the surface of glass fiber fleece is still difficult due to its complex three-dimensional structure and large surface area, in particular as compared to the plane surfaces of filter membranes. The materials used for coating are often hydrophilic polymers. Due to their hydrophilicity, the coating materials do not make a stable contact with the underlying surface of the filter material and may be easily and quickly scrubbed off or leached out. In particular, there is no cross-linking of any these hydrophilic polymeric coating materials to the glass fiber fleece. Hence, the resulting non-cross-linked surface bound hydrophilic polymer coatings are easy to apply but offer limited stability and are prone to leaching.

More robust and longer lasting alternative coatings can be made by in situ polymerization or grafting from polymerization. This way polymeric coatings that are covalently bound and/or cross-linked to the underlying surface

material can be obtained. These coatings possess enhanced stability and provide a long-lasting coating effect. However, the underlying coating process is very complex and time consuming and cannot be easily applied to a three-dimensional structure including inner surfaces as that of glass fiber fleece. Moreover, the necessary and currently available polymerization techniques are often difficult to control at a production scale and in particular difficult to scale to high production numbers. In contrast, the cross-linking as performed by the present invention offers a simple but adaptable coating process. The cross-linking conditions can be adapted by the skilled artisan as desired. For example, cross-linking temperatures can be varied from about 60 °C to about 200 °C by selecting the appropriate cross-linker component. The resulting structure is a durable three-dimensional polymeric network of the glass fiber fleece and the copolymer cross-linked thereto.

In summary, obtaining cross-linked surface bound hydrophilic polymer coatings up to date has been laborious and involves complex processes; in particular with respect to the coating of three-dimensional glass fiber fleeces there appears to be no easy solution today. Reaction conditions for such coatings are difficult to control because the polymerization has to occur in the presence of a solvent (wet environment). Moreover, it is impossible to avoid clogging of the glass fiber fleece pores such that the resulting coating is very inhomogeneous. Further, it is impossible to clean the obtained polymer, and low molecular weight compounds may reside in the polymer structure or coated fleece which may have adverse effects on the filtration or filtrate.

The technical problem underlying this disclosure may be seen in the provision of means and methods complying with the aforementioned needs. In particular, there is a need to provide improved means and methods for obtaining a long lasting and stable hydrophilic coating on three-dimensional structures such as glass fiber fleeces.

The technical problem is solved by the embodiments characterized in the claims and as described herein below.

Advantageous embodiments which might be realized in an isolated fashion or in any arbitrary combinations are listed in the dependent claims as well as throughout the specification.

Thus, the present invention relates to a method for coating a glass fiber fleece comprising contacting the surface of a glass fiber fleece with a hydrophilic copolymer; and crosslinking the copolymer. Moreover, the present invention relates to a glass fiber filter comprising a glass fiber fleece, wherein the surface of the glass fiber fleece is coated with a hydrophilic copolymer; and to a device for biomedical filter applications comprising the same.

The term "glass fiber fleece" as used herein refers to a nonwoven fiber material. The fibers may be of any type. They may in particular have a certain length, they may be filamentous or endless. The term "glass fiber fleece" specifically refers to any type of nonwoven or undirected fiber structure. The glass fiber fleece is typically characterized by a fibrous three-dimensional structure. More typically, the glass fiber fleece forms a three-dimensional network of fibers with irregular spacing. Due to their structure, glass fiber fleeces commonly have a large surface or large surface area. Specifically, their morphology is irregular. Typical glass fiber fleece structures may include fibrous sheets, randomly dispersed fiber matrices, meshes, nonwoven fabrics and the like.

Typically, the glass fiber fleece is suitable for forming a filter material with a pore size suitable for separating cellular components out of liquid samples of bodily fluid; typically for separating blood cells such as erythrocytes from whole blood preparations. Suitable pore sizes may be in the range of 1 $\mu$m to 10 $\mu$m; specifically in the range of 2 $\mu$m to 8 $\mu$m, or in the range of 3 $\mu$m to 6 $\mu$m.

Suitable commercial glass fiber filter products that may be used in line with the present invention are for example borosilicate glass fiber filter materials having an average pore size of 3 to 6 $\mu$m, such as GF/D type commercially available glass fiber fleece by Whatman or SureWick® glass fiber fleeces available by Millipore Merck and/or similar products.

Typically, the glass fibers of the fleece used in the present invention have a fiber diameter in the nano- to micrometer range, e.g. between 1 nm to 1000 nm. Fiber diameter, pore size and/or fiber structure may be analyzed by techniques known in the art, for example by electron microscopic analysis.

Fleece fibers suitable in the method according to the present invention are made of glass or any type of known glass fiber material. According to the present invention, the glass fiber typically comprises soda-glass, low-alkali glass, borosilic acid glass, or quartz.

The terms "glass fiber filter" and "glass fiber fleece filter" are used interchangeably herein. The glass fiber fleece used in the present invention is typically used as a filter material; in particular for biomedical filter applications as specified elsewhere herein.

The term "coating" as used herein generally refers to a layer of material covering a surface of an object, in particular, the surface of a glass fiber fleece. In the context of the present invention, the term may in particular refer to a hydrophilic polymeric layer established by covalent bonding of a hydrophilic copolymer to a surface; more particularly, to the surface of a glass fiber fleece. In particular, in line with the present invention, the coating is covalently bonded to the surface of the object, more particularly covalently bonded to the surface of a glass fiber fleece. Techniques of establishing a covalent bond between a coating and a surface are known in the art and described according to the present invention in further detail elsewhere herein.

The coating according to the present invention specifically refers to a polymeric hydrogel coating. In particular, said coating has a complex three-dimensional structure, typically being flexible. The structure may particularly form a

three-dimensional network of irregular morphology. The term "hydrogel" refers to a polymer forming a complex hydrophilic polymeric network that has a large capacity for binding water. It is typically water insoluble but is permeable for water. Upon water binding, the volume of the polymer may increase, in other words, the hydrogel is capable of swelling in water. Hydrogels are typically biocompatible and may exhibit biological tissue like mechanical properties. In line with the present invention, the hydrogel typically comprises a hydrophilic copolymer and water. More typically, a hydrogel forms upon crosslinking of the hydrophilic copolymer described elsewhere herein to the surface of a glass fiber fleece. The crosslinking typically establishes intermolecular covalent bonds between the molecule chains of the copolymer and the glass fibers and more typically additional intramolecular covalent bonds between chains of the copolymer. These inter and intramolecular bonds typically generate the hydrophilic complex network forming the hydrogel coating.

It was surprisingly found by the present inventors that by the method according to the invention a three-dimensional coating could successfully be applied to a three-dimensional surface of a glass fiber fleece. It was particularly surprising that the resulting coated material exhibits high protein repellency and excellent filter capacities.

The coating according to the invention may be applied by suitable techniques known in the art. These include for example, spraying, spin-coating, dip-coating, doctor blading, immersing or submerging the object to be coated into a coating solution. These techniques typically expose the surface to be coated to a coating formulation or a solution of the coating material. Specifically in line with the present invention, the coating may be applied by submerging a glass fiber fleece into a solution of a hydrophilic copolymer. Application of the coating by submerging into a solution of the copolymer appears advantageous, as it may access large parts, i.e. a large area, of the surface of the fleece material and may cover also inner surfaces that cannot be easily covered by other methods. The terms "submerging" and "immersing" are used interchangeably herein. The application is described in further detail elsewhere herein.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

The method according to the invention comprises a step (a) of contacting the surface of a glass fiber fleece with a hydrophilic copolymer.

The glass fiber fleece material typically has a large surface or large surface area due to its three-dimensional structure formed by nonwoven undirected fibers. These fibers particularly form a large surface including inner surfaces that increase the overall surface area. Typically, the term "surface of a glass fiber fleece" refers to the overall surface area and hence may reflect the sum of outer and inner surface area. More typically, the surface area of the glass fiber fleece may be estimated by determining the specific surface area. The specific surface area can be measured and determined by techniques known in the art. Typically, the volume specific surface area can be calculated based on the occupied volume, the free volume and the volume of the fibers. The volume specific surface area $S_v$ (also referred to as $O_{spez}$) may typically be calculated based on the following equation, wherein A is the surface area and V is the occupied volume:

$$S_v = A/V \ [m^2/m^3]$$

Further calculations of $S_v$ (also referred to as $O_{spez.}$) are given in the examples below.

In the method according to the invention, the coating solution advantageously can access the inner surface area in the step of submerging the glass fiber fleece into the solution of the hydrophilic copolymer. This way, the entire three dimensional structure of the glass fiber fleece is thought to be soaked by the copolymer solution. Thereby, also the inner

surface area can be reached. Moreover, the copolymeric coating can be crosslinked to the inner surface area of the glass fiber fleece establishing a stable covalent bonding. Hence, the method of the present invention is advantageous as it provides a way of establishing a durable coating on the entire surface of a glass fiber fleece, including the inner surface area or at least parts thereof.

The method according to the present invention comprises step (a) of contacting the glass fiber with a solution of the copolymer; specifically with a low concentration of said copolymer. More specifically, step (a) of contacting comprises contacting the glass fiber with a solution containing 0.1 to 10 mg/mL of the copolymer; typically 0.2 to 2 mg/mL of the copolymer. The described concentration is advantageous as although in the low range it is surprisingly already sufficient to establish a stable and durable coating with excellent protein repellent properties also on the inner surface.

Typically, the hydrophilic copolymer can be dissolved in an appropriate solvent to obtain a solution thereof. A suitable solvent for the copolymer in line with the present invention may be a polar solvent, typically an aqueous buffer solution, ethanol or water. The hydrophilic copolymer for use in the present invention in particular is water-soluble or at least partially water-soluble. It is more particularly capable of forming a hydrogel; still more particularly, it may form a hydrogel on the three-dimensional surface of a glass fiber fleece.

The hydrophilic copolymer specifically comprises at least first a monomer and at least a second monomer. More specifically, the hydrophilic copolymer comprises at least first a monomer comprising an acrylate or an acrylamide, even more specifically an N-alkyl acrylamide, and at least a second monomer comprising a crosslinker component. Still more specifically, the hydrophilic copolymer consists of a first a monomer comprising an acrylate or an acrylamide and a second monomer comprising a crosslinker component. In a particular copolymer of the invention, a typical crosslinker content ranges from 0.1 to 25 mol%, more typically the crosslinker content ranges from 0.5 to 10 mol%. The specified ranges are advantageous due to the following effects: at higher crosslinker concentration, the water swellability of the forming polymeric network is strongly attenuated due to the hydrophobic nature of the crosslinker units and at very low concentrations, the resulting hydrogels become mechanically fragile.

Typically, the first monomer is or consists of an acrylate or an acrylamide. More typically, the acrylamide is selected from the group consisting of acrylamide, *N*-methyl acrylamide, *N,N*-dimethyl acrylamide, *N*-ethyl acrylamide, *N*-propyl acrylamide, *N*-butyl acrylamide, and *N,N*-diethyl acrylamide; and the acrylate is selected from 2-hydroxymethylacrylate and 2-hydroxyacrylate.

Typically, the second monomer is or consists of a crosslinker component. The term "cross-linker" as referred to herein typically relates to a chemical component that upon activation is capable of forming a reactive intermediate such as a radical. More typically, the cross-linker can be activated thermally or by photo-activation. The term "activation" as used herein typically relates to a treatment resulting in the formation of a reactive intermediate. Hence, thermal activation refers to heat treatment while photo-activation refers to treatment with UV irradiation. Both treatments lead to the formation of the reactive intermediate.

Specifically, the crosslinker component is a crosslinker selected from the group consisting of azides and diazo group containing crosslinkers. A typical azid crosslinker useful in the present invention is a sulfonyl azide such as 4-styrenesulfonyl azide or 4-propylbenzenesul-fonyl azide. Alternatively, the crosslinker may be a diazo group containing crosslinker selected from the group consisting of: 2-(2-diazo-2-phenylacetoxy)ethylmethacrylate, or 1-(4-(methacryloyloxy)butyl)-3-methyl-2-diazomalonate. The reactive intermediate that is formed from said crosslinker is typically a carbene or a nitrene radical.

According to the present invention, the hydrophilic copolymer may be represented by the following formula, formula A:

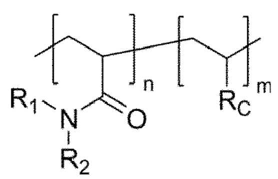

wherein $R_1$ and $R_2$ are independently from each other selected from H, $CH_3$, $C_2H_5$, $C_3H_7$. Rc respresents the crosslinker component. Rc may typically be selected from:

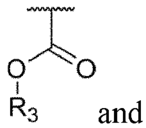

and

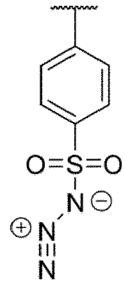

Typically, n and m represent a number between 1 and 2000. More typically, n is between 10 and 1000 and/or m is between 5 and 100. Specifically, the copolymer is a statistical copolymer. In other words, specifically the monomers are statistically distributed in the copolymer. More specifically, n has a number that is larger than m. Still more specifically, the number of n is at least two times the number of m: even more specifically the number of n is at least three, at least four, at least five, at least ten times the number of m. In a typical copolymer according to the invention, n and m represent a number between 1 and 2000, typically n is between 10 and 1000 and/or m is between 5 and 100.

$R_3$ is typically an azide or diazo group containing residue; more typically $R_3$ is selected from:

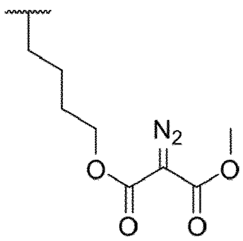

and

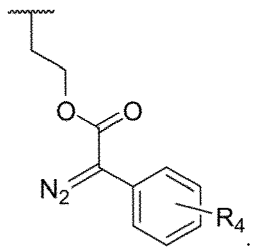

.

$R_4$ is typically selected from H, OH, F, Cl, $CH_3$, $C_2H_5$, $C_3H_7$; more typically, $R_4$ is H or $CH_3$, even more typically H.

Typically, n and m represent a number between 1 and 2000. More typically n is between 10 and 1000 and/or m is between 5 and 100.

More specifically, the hydrophilic copolymer according to the present invention may be represented by formula I:

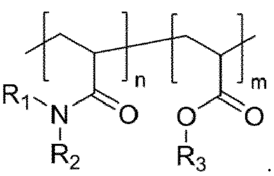

.

Typically $R_1$, $R_2$ and $R_3$ are as defined above.

According to the present invention, the hydrophilic copolymer may alternatively, be represented by the following formula, formula II:

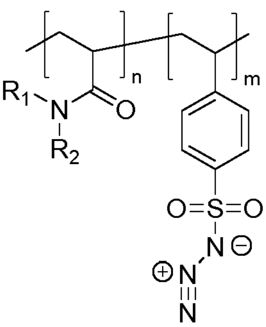

In formula II, $R_1$ and $R_2$ are, as in formula I, typically independently from each other selected from H, $CH_3$, $C_2H_5$, $C_3H_7$. The numbers for n and m are as in formula I.

Typically, n and m represent a number between 1 and 2000. More typically n is between 10 and 1000 and/or m is between 5 and 100.

The crosslinker component can be synthesized as known in the art and exemplified elsewhere herein.

The copolymer can be obtained via conventional free-radical copolymerization with co-monomers that typically form water soluble polymers. More typically, the copolymer forms a hydrogel. In particular, the copolymer can be obtained via free-radical copolymerization using as co-monomers, the at least first and at least second monomer as specified elsewhere herein. As a polymerization initiator common radical initiators may be used, typically 2,2'-azobis(2-methylpropionitril) (AIBN) or the like. By varying the co-monomer feed, typically copolymers with crosslinker contents between 0.1 to 25 mol%, more typically between 0.5 and 10 mol%, may be generated. Still more typically, the hydrophilic copolymer comprises at least a content of 2.5 mol% of crosslinker component, typically at least 5 mol% of cross-linker component. The content of crosslinker within the copolymer can be determined by NMR analysis, e.g. $^{1}$H-NMR analysis. The respective means and methods are well known in the art. Further details are described in the example section below.

Typical examples for a copolymer according to the present invention are represented by the following structural formulas:

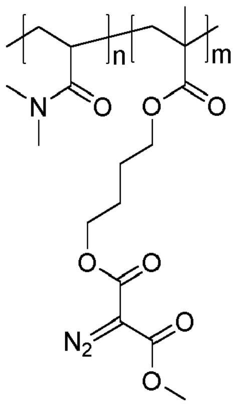

p(DMMA-MAz)

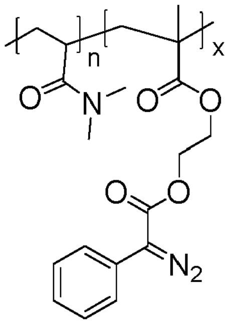

p(DMMA-PEDAZ)

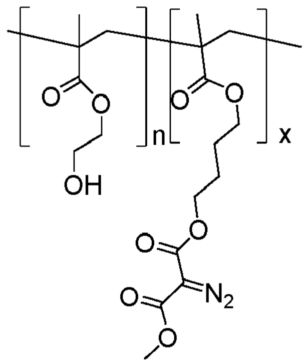

p(HEMA-MAz).

In the method according to the invention, the glass fiber fleece is contacted with the hydrophilic copolymer typically as a first step in the coating process. The contacting step may refer to any way of applying or coating the copolymer solution onto the glass fiber surface including spin or dip coating, spraying, submerging or doctor blading. In order to maximize the contact area between the surface of the glass fiber fleece and the hydrophilic copolymer solution, the contacting may more specifically refer to submerging the glass fiber fleece in the solution of the copolymer. Typically, the submerging is performed for a time suitable for wetting or soaking the entire surface area of the glass fiber fleece. More typically, the submerging is performed for at least 30 min, still more typically for at least 45 min, even more typically for at least 60 min. Submerging may take place for a up to 2 h. During submerging, the glass fiber fleece may typically be constantly shaken to ensure complete wetting of the entire surface area of the glass fiber fleece. Submerging combined with shaking has the advantageous effect that the contact area between the hydrophilic copolymer solution and the surface of the glass fiber fleece may be maximized. Thereby, ideally an essentially completely soaked glass fiber fleece can be obtained.

Subsequently, the method may comprise a step of removing residual solvent that may arise from the hydrophilic copolymer solution. For removing residual solvent, typically, the glass fiber fleece is removed from the copolymer solution and the solvent is evaporated at a temperature of 50 °C or above. Specifically, the solvent may be removed at a temperature of 60 °C or above. More specifically, the temperature shall not exceed 100 °C. A temperature range of between 50 °C and 100 °C is specifically suitable as the solvent may be completely evaporated while the crosslinking reaction will not be started.

Moreover, the method according to the invention comprises a step of crosslinking the copolymer. Typically, the crosslinking step establishes a covalent bond between the copolymer and the glass fiber fleece. The term "crosslinking" as used herein generally refers to a chemical reaction establishing a link or chemical bond between two different molecules or molecule chains, in particular polymer chains. In line with the present invention, the term "cross-linking" in particular refers to establishing a chemical bond between the glass fiber fleece and the hydrophilic copolymer. The chemical bond established by the crosslinking is typically a covalent bond. A covalent bond is advantageous as it establishes a stable, durable and long lasting coating of the copolymer on the surface of the glass fiber fleece.

The crosslinking step of the method of the present invention typically comprises a C-H insertion mechanism; specifically the crosslinking occurs by a C-H insertion mechanism. This is also referred to as C-H insertion reaction and specified elsewhere herein in more detail.

The crosslinking in the method of the present invention, in particular refers to establishing a surface-attached

hydrophilic copolymer network on the surface of the glass fiber fleece. Thereby, advantageously a copolymeric network is created on the three-dimensional structure of the glass fiber fleece surface that even more increases the overall inner surface area. The hydrophilic copolymeric coating may also be referred to as a hydrogel or hydrogel coating.

Typically, the described crosslinking step establishes the surface-attached polymer network. More typically, the crosslinking establishes a covalent bond between the copolymer and the glass fiber fleece thereby creating a surface-attached polymer network on the glass fiber surface. The resulting coated glass fiber fleece surprisingly has excellent protein repellent properties in particular in blood filter applications.

In particular, in the crosslinking step (step (b)) the copolymer is covalently bonded to the glass fiber by a C-H insertion reaction or C-H insertion mechanism.

The C-H insertion reaction and methodology for stable surface-attached polymeric coatings has recently been established in the art and is for example reviewed in Prucker et al. (Biointerphases, Vol. 13, No. 1, Jan/Feb 2018) and Kotrade and Rühe (Angew. Chem. Int. Ed. 2017, 56, 14405 -14410). The reaction relies on the incorporation of groups with latent reactivity, herein designated as a crosslinker component, as pendant co-monomers into the copolymer to be deposited. In brief, in the art the respective copolymers are, in a first step, applied to the surface of an object to be coated using standard coating techniques such as spin or dip coating, spraying, or doctor blading. A reactive intermediate forms upon thermal or photo-activation from the crosslinker, such as a nitrene or a carbene. The reactive intermediate can insert into a C-H bond of the surface material, establishing a covalent bond between the copolymer and the surface of the object. However, up to date, the methodology has been successfully used only for coating flat surfaces but not to three-dimensional structures such as the surface of glass fiber fleece. It was generally assumed in the art, that applying a polymeric coating to a three-dimensional structure such as a glass fiber fleece would clog or reduce the free space in the three-dimensional network and hence would lead to a reduction in the filter capacities of the coated material. The present inventors surprisingly found that contrary to the general assumption, the filter capacities were excellent and the protein repellency was superior in the resulting copolymer coated glass fiber fleece material.

According to the method of the present invention, the crosslinking in step (b) typically comprises heating the copolymer contacted with the glass fiber to at least 40 °C, specifically to at least 50 °C; more specifically to a temperature between 50 °C and 200 °C.

As noted elsewhere herein, the crosslinker may typically be activated by thermal activation, i.e. by applying thermal energy or heat to the copolymer. The application of thermal energy is in particular performed in an oven set to an appropriate temperature. However, other routes of heating the copolymer may be envisaged such as pressure application. Alternatively, the crosslinker may be photo-activated such as by UV- irradiation. However, thermal activation is regarded as being advantageous for coating of surfaces with a three-dimensional structure such as glass fiber fleece since it may also reach the inner surface area and allow for a cross-linking reaction there.

The heating step, i.e. the step for thermal activation, is typically performed for at least 15 minutes, typically at least 20 min, more typically for at least 20 minutes up to 2 hours; even more typically between 20 min and 1 h. The time required for thermal activation may vary depending on the temperature applied. At a lower temperature, such as 120 °C, 1h of activation time may be needed for sufficient crosslinking, while at a temperature of 160 °C 30 min may be already sufficient for achieving an efficient crosslinking.

The method of the invention may comprise any or all of the following additional steps:

- washing the glass fiber fleece; and/or
- drying the glass fiber fleece, and/or
- obtaining a copolymer network coated glass fiber fleece.

One typical method in line with the present invention may have the following steps:

(a) contacting the surface of a glass fiber fleece with a hydrophilic copolymer;
(b) crosslinking the copolymer; and
(c) obtaining a coated glass fiber fleece.

Still typically, in step (a) the contacting comprises submerging the glass fiber fleece in a solution of the hydrophilic copolymer in a suitable solvent; the submerging may optionally be accompanied by constantly shaking the submerged glass fiber fleece; further optionally the submerging may be followed by evaporating the solvent. Moreover the method may typically involve a step of washing and/or drying the coated glass fiber fleece typically after the crosslinking (b) is performed; and prior to obtaining the coated glass fiber fleece (step (c)).

The glass fiber fleece coated in line with the present invention is particularly suitable as filter material in biomedical filter applications, typically blood filtration; more typically removal of blood cells, in particular erythrocytes, from whole blood preparations.

Moreover, the present invention relates to a glass fiber filter, comprising a glass fiber fleece, wherein the surface

of the glass fiber fleece is coated with a hydrophilic copolymer network.

The glass fiber filter according to the present invention is coated is coated with a hydrophilic copolymer or copolymer network as specified elsewhere herein; specifically, the coating comprises a covalent bonding between the copolymer and the glass fiber.

Further, the present invention contemplates a device for biomedical filter applications, typically blood filtration, comprising a glass fiber filter as specified elsewhere herein.

Still further, the present invention contemplates a method for separating plasma from blood, comprising filtering the blood with a glass fiber filter or device according to the invention.

The present invention further relates to the use of a hydrophilic copolymer for coating a glass fiber fleece.

Summarizing and without excluding further possible embodiments, the following embodiments may be envisaged:

1. A method for coating a glass fiber fleece comprising the following steps:

(a) contacting the surface of a glass fiber fleece with a hydrophilic copolymer; and
(b) crosslinking the copolymer.

2. The method according to embodiment 1, wherein the copolymer comprises at least first a monomer comprising an acrylate or an acrylamide, specifically of an N-alkyl acrylamide, and at least a second monomer comprising a crosslinker component.

3. The method according to any of the preceding embodiments, wherein the crosslinking comprises a C-H insertion mechanism; specifically the crosslinking occurs by a C-H insertion mechanism.

4. The method according to the preceding embodiment, wherein the C-H insertion is achieved by thermal activation or activation via UV irradiation.

5. The method according to any of the preceding embodiments, wherein the hydrophilic copolymer is represented by the following formula A:

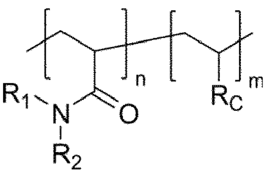

wherein $R_1$ and $R_2$ are independently from each other selected from H, $CH_3$, $C_2H_5$, $C_3H_7$; and wherein Rc respresents the crosslinker component.

6. The method according to the preceding embodiment, wherein Rc is selected from:

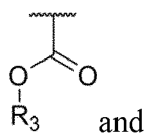
and
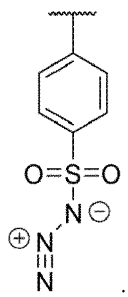
.

7. The method according to any of the preceding embodiments, wherein the hydrophilic copolymer is represented by the following formula I:

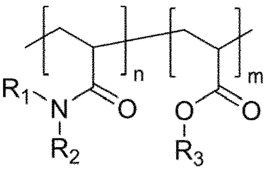

wherein $R_1$ and $R_2$ are independently from each other selected from:

H, $CH_3$, $C_2H_5$, $C_3H_7$;
and wherein $R_3$ is selected from an azide or diazo group containing residue.

8. The method according to any of the preceding embodiments, wherein the acrylamide is selected from the group consisting of: acrylamide, *N*-methyl acrylamide, *N,N*-dimethyl acrylamide, *N*-ethyl acrylamide, *N*-propyl acrylamide, *N*-butyl acrylamide, and *N,N*-diethyl acrylamide; and wherein the acrylate is selected from 2-hydroxymethylacrylate and 2-hydroxyacrylate.

9. The method according to any of the preceding embodiments, wherein the crosslinker component is a sulfonyl azide, such as 4-styrenesulfonyl azide or 4-propylbenzenesul-fonyl azide.

10. The method according to any of the preceding embodiments, wherein the crosslinker component is selected from the group consisting of: 2-(2-Diazo-2-phenylacetoxy)ethylmethacrylate, or 1-(4-(Methacryloyloxy)butyl)-3-methyl-2-diazomalonate.

11. The method according to any of the preceding embodiments, wherein $R_3$ is a residue selected from the group consisting of:

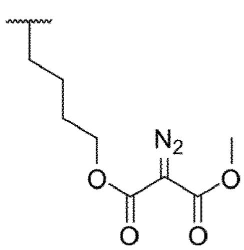 and 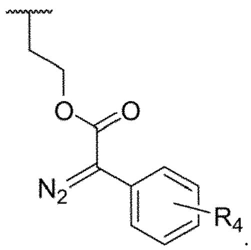.

12. The method according to the preceding embodiment, wherein $R_4$ is selected from H, OH, F, Cl, $CH_3$, $C_2H_5$, $C_3H_7$, typically, $R_4$ is H or $CH_3$, more typically H.

13. The method according to any of the preceding embodiments, wherein the hydrophilic copolymer is represented by the following formula II:
wherein $R_1$ and $R_2$ are independently from each other selected from:

H, $CH_3$, $C_2H_5$, $C_3H_7$ ;and wherein n and m represent a number between 1 and 2000,
typically n is between 10 and 1000 and/or m is between 5 and 100.

14. The method according to any of the preceding embodiments, wherein Rc is selected from:

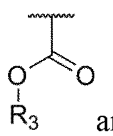 and 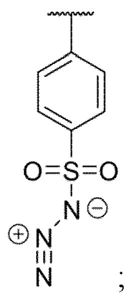 ;

wherein $R_3$ is a residue selected from:

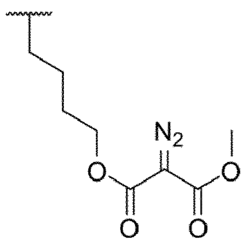 and 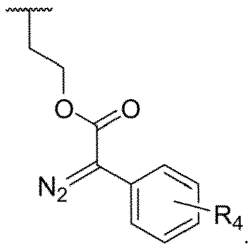

wherein $R_4$ is typically selected from H, OH, F, Cl, $CH_3$, $C_2H_5$, and $C_3H_7$.

15. The method according to any of the preceding embodiments, wherein the hydrophilic copolymer comprises at least a content of 2.5 mol % of crosslinker component, typically at least 5 mol% of crosslinker component.

16. The method according to any of the preceding embodiments, wherein step (b) comprises heating the copolymer contacted with the glass fiber to at least 40 °C, specifically to at least 50 °C; more specifically to a temperature between 50 °C and 200 °C.

17. The method according to the preceding embodiment, wherein the heating is performed for at least 15 minutes, typically at least 20 min, more typically for at least 20 minutes to 2 hours.

18. The method according to any of the preceding embodiments, further comprising:

(c) washing the glass fiber fleece; and/or
(d) drying the glass fiber fleece.

19. The method according to any of the preceding embodiments, further comprising a step of obtaining a copolymer network coated glass fiber.

20. The method according to any of the preceding embodiments, wherein the glass fiber comprises soda-glass, low-alkali glass, borosilic acid glass, or quartz.

21. The method according to any of the preceding embodiments, wherein the glass fiber filter is a glass fiber filter suitable for biomedical filter applications, typically blood filtration.

22. The method according to any of the preceding embodiments, wherein the step (a) of contacting comprises contacting the glass fiber with a solution of the copolymer; specifically with a low concentration of the copolymer.

23. The method according to any of the preceding embodiments, wherein the step (a) of contacting comprises contacting the glass fiber with a solution containing 0.1 to 10 mg/mL of the copolymer; typically 0.2 to 2 mg/mL of the copolymer.

24. A glass fiber filter, comprising a glass fiber fleece, wherein the surface of the glass fiber fleece is coated with a hydrophilic copolymer, specifically with a hydrophilic copolymer network, specifically with a hydrogel.

25. The glass fiber filter according to the preceding embodiment, wherein the coating comprises a covalent bonding between the copolymer and the glass fiber.

26. The glass fiber filter according to the preceding embodiment, wherein the copolymer comprises at least a content of 2.5 mol% of crosslinker component, typically at least 5 mol% of crosslinker component.

27. A device for biomedical filter applications, typically blood filtration, comprising a glass fiber filter according to any of the preceding embodiments.

28. A method for separating plasma from blood, comprising filtering the blood with a glass fiber filter or device according to any of the preceding embodiments.

29. Use of a hydrophilic copolymer for coating a glass fiber fleece.

**Short description of the Figures**

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.
In the Figures:

**Figure 1** shows a bar representation of the recovery rate of Troponin T for the untreated fleece (UB), the plasma separation fleece as used in the Roche CARDIAC test strip family (e.g. Roche CARDIAC Troponin T, Roche CARDIAC proBNP, Roche CARDIAC D-Dimer) (C), and the fleece coated with p(DMAA-MAz) according to the invention. The coating was performed by submerging the fleece in a solution of 0.1 mg/ml of p(DMAA-MAz) in ethanol. The crosslinking of p(DMAA-MAz) was performed at 160 °C. Depicted are the results retrieved with low cTnT concentration at 170 pg/ml and high cTnT concentration at 1700 pg/ml.

**Figure 2** shows a bar representation of the recovery rate of Troponin T for the plasma separation fleece as used in the Roche CARDIAC test strip family (e.g. Roche CARDIAC Troponin T, Roche CARDIAC proBNP, Roche CARDIAC D-Dimer) (Cardiac), and the fleece coated with p(HEMA-MAz) according to the invention (I). The coating was performed by submerging the fleece in a solution of 2 mg/ml of p(HEMA-MAz) in ethanol. The crosslinking of p(HEMA-MAz) was performed at 160 °C. Depicted are the results retrieved with low cTnT concentration at 100 pg/ml and high cTnT concentration at 1000 pg/ml.

**Figure 3** shows a bar representation of the recovery rate of Troponin T for the plasma separation fleece as used in the Roche CARDIAC test strip family (e.g. Roche CARDIAC Troponin T, Roche CARDIAC proBNP, Roche CARDIAC D-Dimer) (C), and the fleece coated with p(DMMA-PEDAZ) according to the invention (I). The coating was performed by submerging the fleece in a solution of 2 mg/ml of p(DMAA-PEDAZ) in ethanol. The crosslinking of p(DMMA-PEDAZ) was performed at 100 °C. Depicted are the results retrieved with low cTnT concentration at 100 pg/ml and high cTnT concentration at 1000 pg/ml.

**Figure 4** is a line graph of the results of a lateral flow assay for different Troponin T concentrations. Plotted are the [%] remission values for a glass fiber fleeces coated with p(DMMA-MAz) and crosslinked at either 120 °C for 60 min or 160 °C for 30 min and for the commercially available plasma separation fleece as used in the Roche CARDIAC test strip family (e.g. Roche CARDIAC Troponin T, Roche CARDIAC proBNP, Roche CARDIAC D-Dimer).

**Figure 5** shows a bar representation of the recovery rate of Troponin T for different samples of glass fiber fleece coated with p(DMAA-MAz) immersed in different concentrations of the polymer (from 0.1 to 10 mg/ml). All samples were cross-linked at 160°C for 30min. Depicted are the results retrieved with low cTnT concentration at 170 pg/ml and high cTnT at concentration 1700 pg/ml.

**Figure 6** shows a bar representation of the recovery rate of Troponin T for a BSA coated glass fiber fleece and a glass fiber fleece coated with p(DMAA-MAz). Depicted are the results retrieved with low cTnT concentration at 170 pg/ml and high cTnT concentration 1700 pg/ml.

**Examples**

**Example 1: Synthesis of p(DMAA-MAz)**

The diazomalonic ester monomer (MAz) was obtained following the reaction pathway as known in the art and described e.g. in Kotrade and Rühe (Angew. Chem. Int. Ed. 2017, 56, 14405 -14410). In brief, methyl malonyl chloride is transformed into 4-hydroxybutyl methyl malonate, followed by esterification with methacryloyl chloride and subsequent functionalization of the C-H acidic malonate group through a Regitz diazo-transfer reaction. For the Regitz reaction an azide was used to transfer a diazo group to the target molecule to yield the polymerizable diazomalonic ester (MAz).
The synthesis of (pDMMA-MAz), a statistical copolymer with the hydrophilic main (first) monomer dimethyl acrylamide (DMMA) and the crosslinker component (second monomer) 1-(4-(methacryloyloxy)butyl)-3-methyl-2-diazomalonate (MAz), was achieved by free-radical polymerization at 60 °C. 2,2'-azobis(2-methylpropionitril) (AIBN) was used as initiator.

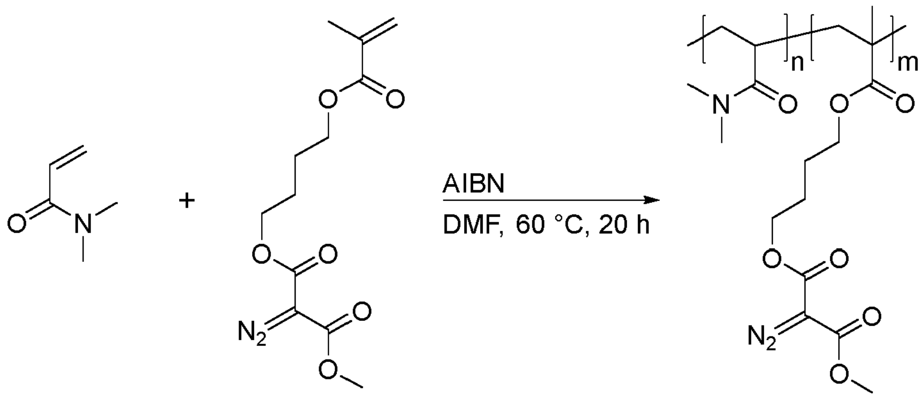


1 2 3

Formula II p(DMAA-MAz)

For synthesizing p(DMMA-MAz) a dried Schlenk tube was prepared. The monomers MAz and DMMA were added to the tube and dissolved in DMF. The initiator AIBN was also dissolved in DMF and added to the tube. Dissolved gases were removed by repeated cycles (6 times) of freezing the solution by liquid nitrogen, and thawing under vacuum.

The solution was polymerized under vacuum application overnight at 60 °C in a water bath. Thereafter, the copolymer was precipitated in 300 ml diethylether; resolved in chloroform and again precipitated in 300 ml diethylether. Drying was performed overnight by application of a high vacuum. The copolymer was obtained as a white solid substance.

The obtained copolymer was analyzed to determine the crosslinker content, and other molecular characteristics

**$^1$H-NMR:** (250 MHz, $CDCl_3$): δ (ppm) = 4,5-3,7 (m, 7 H, $H_3COOR$, $RH_2COOR$), 3,3-0,5 ppm (m, 10H + polymer backbone, $H_3C$-NCOR, CH, $CH_2$, $CH_3$).

**Gel Permeation Chromatography (GPC):**

**Tab. 1:** GPC data of obtained P(DMAA-X %MAz).

| Crosslinker content [mol%] | $M_n$ [g/mol] | $M_w$ [g/mol] | Poly Dispersion Index (PDI) |
|---|---|---|---|
| 7,5 % Batch 1 | 182.000 | 592.000 | 3,2 |
| 7,5 % Batch 2 | 34.300 | 116.000 | 3,4 |
| 15% MAz | 53.000 | 258.000 | 4,9 |
| 20 % MAz | 101.000 | 390.000 | 3,8 |

**Example 2: Synthesis of the p(HEMA-MAZ)**

The synthesis was analogous to example 1 above. However, the main monomer was 2-hydroxyethylmethacrylate (HEMA). In the coating process, the glass fiber fleece as submerged in a solution of the copolymer of 2 mg/ml. A crosslinker content of 7.5 mol% was determined by $^1$H-NMR.

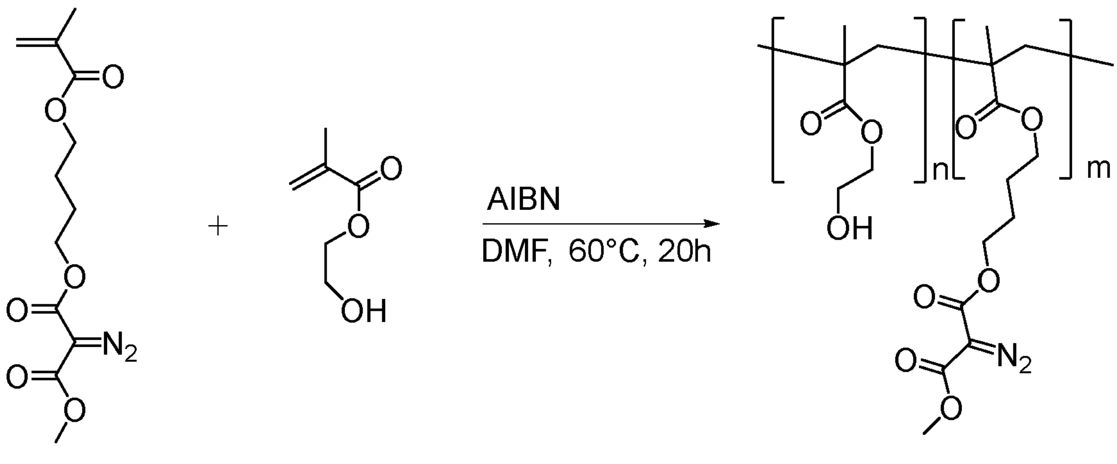


1 2 3

For synthesizing p(HEMA-MAz) a dried Schlenk tube was prepared. The monomers MAz and HEMA were added to the tube and dissolved in DMF. The initiator AIBN was also dissolved in DMF and added to the tube. Dissolved gases were removed by repeated cycles (4 times) of freezing the solution by liquid nitrogen, and thawing under vacuum.

The solution was polymerized under vacuum application overnight at 60 °C in a water bath. Thereafter, the copolymer was precipitated in 300 ml diethylether; resolved in chloroform and again precipitated in 300 ml diethylether. Drying was performed overnight by application of a high vacuum. The copolymer was obtained as a white solid substance.

**$^1$H-NMR:** (250 MHz, $CDCl_3$): $\delta$ (ppm) = 4,80 ppm (s, 2 H, $H_2COOR$), 4,1-3,5 ppm (m, 9 H, $H_3COOR$, $RH_2COOR$, $H_2OH$), 2-0,5 ppm (m, 6H + polymer backbone, $H_3C$-NCOR, $CH_2$, $CH_3$).

**Example 3: Synthesis of the P(DMAA-PEDAZ)**

The synthesis was analogous to example 1 above. However, the crosslinker group was 2-(2-diazo-2-phenylacetoxy)ethyl methacrylate (PEDAZ). In the coating process, the glass fiber fleece as submerged in a solution of the copolymer of 2 mg/ml. A crosslinker content of 5 mol% was determined by $^1$H-NMR.

For synthesizing p(DMMA-PEDAZ) a dried Schlenk tube was prepared. The monomers PEDAZ and DMMA were added to the tube and dissolved in DMF. The initiator AMDVN was also dissolved in DMF and added to the tube. Dissolved gases were removed by repeated cycles (4 times) of freezing the solution by liquid nitrogen, and thawing under vacuum.

The solution was polymerized under vacuum application overnight at 30 °C in a water bath. Thereafter, the copolymer was precipitated in 300 ml diethylether; resolved in chloroform and again precipitated in 300 ml diethylether. Drying was performed overnight by application of a high vacuum. The copolymer was obtained as a white solid substance.

The obtained copolymer was analyzed to determine the crosslinker content, and other molecular characteristics

**$^1$H-NMR:** (250 MHz, $CD_2Cl_2$): $\delta$ (ppm) = 7,6-7 ppm (m, 5H, Ar-H), 4,6-4 ppm (m, 4 H, $H_2COOR$), 3,5-0,5 ppm (m, 6H + polymer backbone, $H_3C$-NCOR, CH, $CH_2$, $CH_3$).

GPC:

Mn= 85.000 g/mol
Mw= 197.000 g/mol
PDI: 2.32

**Example 4: Coating a glass fiber fleece**

<u>Submerging</u>

The glass fiber fleece was submerged in a solution of the copolymer in ethanol at a copolymer concentration of 0.1 mg/ml for 1 h under constant shaking. It is thought that the shaking ensures complete soaking of the copolymer solution into the fiber structure of the glass fiber fleece. Thereafter, the solvent was evaporated at a minimum temperature of 60 °C for at least 3 h.

<u>Crosslinking</u>

Thermal crosslinking was performed for 30 min at 160 °C in an oven. Thereby the C-H insertion reaction was

initiated.

Washing

After crosslinking, the glass fiber fleece was washed for 2h in ethanol to remove non-cross-linked copolymer from the glass fiber fleece. After washing the coated glass fiber fleece was dried to evaporate the solvent at a minimum temperature of 60 °C for at least 3 h.

**Example 5: Experimental analysis of the coated glass fiber fleece - troponin T recovery**

Protein repellent effect of a p(DMMA-MAz) coating

To assess the protein repellent effect of the coated glass fiber fleece, blood sera of known Troponin T concentrations were centrifuged through the coated glass fiber fleece, commercially available cardiac fleece and non coated glass fiber fleece as a control. After centrifugation the Troponin T concentration is evaluated using a cobas e 601 instrument (Roche Diagnostics GmbH) and compared to the original concentration to evaluate the level of protein adsorption to the different fleece materials (see Fig. 1)

The concentration of troponin T was reduced to about 30 -40 % for cardiac fleece or uncoated fleece. In comparison, the p(DMMA-MAz)_coated glass fiber fleece according to the invention retained 85-90 % of the original troponin T concentration.

Protein repellent effect of a p(HEMA-MAz) coating

The analysis was performed as for p(DMMA-MAz) coating above.

The concentration of troponin T was reduced to about 50 -60 % for cardiac fleece. In comparison the p(HEMA-MAz) coated glass fiber fleece according to the invention retained 80 % of the original troponin T concentration (see Fig. 2).

Protein repellent effect of a p(DMMA-PEDAZ) coating

The analysis was performed as for p(DMMA-MAz) coating above.

The concentration of troponin T was reduced to about 55 -60 % for cardiac fleece. In comparison the p(DMMA-PEDAZ) coated glass fiber fleece according to the invention retained about 90 % of the original troponin T concentration (see Fig. 3).

**Example 6: Experimental analysis of the coated glass fiber fleece -Lateral flow** assay The coating of glass fiber fleece material was prepared as described in examples 1 and 4 above (p(DMMA-MAz) with the only difference that the crosslinking was performed at the above described temperature of 160 °C for 30 min or at a reduced temperature of 120 °C for 60 min.

The different fleece materials were analyzed in a lateral flow assay (LFA) for troponin T. Blood samples of known troponin T concentration were applied to the LFA and quantitatively analyzed in a cobas h232 device (Roche Diagnostics). A low remission signal corresponds to a high single intensity. Both of the glass fiber fleece materials coated in line with the invention showed a better signal intensity than the commercially available cardiac fleece (see Fig. 4).

**Example 7: Experimental analysis of glass fiber fleece coated using different copolymer concentrations**

The coating of glass fiber fleece material was prepared as described in examples 1 and 4 above (p(DMMA-MAz) with the only difference that the coating of the glass fiber fleece material was performed using different p(DMMA-MAz) concentrations, 0.1, 1 or 10 mg/ml. The glass fiber fleece material was assayed for troponin T recovery as described in example 5 above. The results are depicted in Fig. 5.

**Example 8: Troponin T recovery compared to BSA coated glass fiber fleece**

The coating of glass fiber fleece material was prepared as described in examples 1 and 4 above (p(DMMA-MAz) with the only difference that the coating of the glass fiber fleece material was performed using either 2mg/ml p(DMMA-MAz) or 2 mg/ml BSA.

The glass fiber fleece material was assayed for troponin T recovery as described in example 5 above. The results are depicted in Fig. 6.

**Example 9: Analysis of the glass fiber fleece structure**

The density of the glass fiber fleece material was estimated by calculating the volume and determining the weight of a given piece of glass fiber material. The density was estimated to be around 0.13 $g/cm^3$. This represents a density in line with the recommendation for a glass fiber fleece for whole blood separation. Based on the density of glass of 2.5 $g/cm^3$ a free volume of 95 % was calculated.

For characterizing the fiber structure of the glass fiber fleece a scanning electron microscope was used. It could be seen that the fiber structure was undirected and there were smaller and larger areas of free space between individual fibers. The diameter of the fiber was non-uniform but varied in the range of below 1 μm to more than 5 μm.

Assuming a length of 1 mm and a diameter of 2 μm for a glass fiber in the fleece material, the volume of an individual fiber was calculated based on the following equation:

$$V_F = \pi \cdot l \cdot (d/2)^2;$$

resulting in $V_F \approx 3 \cdot 10^{-9}$ $cm^3$.

At a free volume of 95 %, the total volume $V_{Ges}$ is given by:

$$V_{Ges} = 20\ V_F;$$

resulting in $V_{Ges} \approx 6 \cdot 10^{-8}$ $cm^3$.

The surface area of a fiber ($O_F$) was calculated using $O_F = \pi \cdot d \cdot l$ ; resulting in $O_F \approx 6 \cdot 10^{-5}$ $cm^2$.

Based on the foregoing, a volume specific surface area ($O_{spez.}$) was determined: $O_{spez.} = O_F/V_{Ges}$ ; resulting in $O_{spez.} \approx 1000$ $cm^2/cm^3$.

In summary, the glass fiber fleece has a large surface area with an irregular morphology. Such structures are expected to lead to undesired structural effects during coating and to result in unwanted adsorption of proteins; in particular blood proteins during whole blood separation.

Literature cited

Kotrade and Rühe, Malonic Acid Diazoesters for C-H Insertion Crosslinking (CHic) Reactions: A Versatile Method for the Generation of Tailor-Made Surfaces; Angew. Chem. Int. Ed. 2017, 56, 14405 -14410

Prucker et al., Surface-attached hydrogel coatings via C,H-insertion crosslinking for biomedical and bioanalytical applications (Review); Biointerphases, Vol. 13, No. 1, Jan/Feb 2018

EP0457183 A1

EP1618940 A1

JP2002350428 A

**Claims**

1. A method for coating a glass fiber fleece comprising the following steps:

   (a) contacting the surface of a glass fiber fleece with a hydrophilic copolymer; and
   (b) crosslinking the copolymer.

2. The method according to claim 1, wherein the copolymer comprises at least a first monomer comprising an acrylate or an acrylamide, specifically an N-alkyl acrylamide, and at least a second monomer comprising a crosslinker component.

3. The method according to any of the preceding claims, wherein the crosslinking comprises a C-H insertion mechanism; specifically the crosslinking occurs by a C-H insertion mechanism.

**4.** The method according to the preceding claim, wherein the C-H insertion is achieved by thermal activation or activation via UV irradiation.

**5.** The method according to any of the preceding claims, wherein the hydrophilic copolymer is represented by the following formula A:

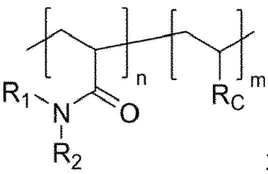
;

wherein $R_1$ and $R_2$ are independently from each other selected from:

H, $CH_3$, $C_2H_5$, $C_3H_7$;
and wherein Rc represents the crosslinker component.

**6.** The method according to any of the preceding claims, wherein the acrylamide is selected from the group consisting of: acrylamide, N-methyl acrylamide, *N,N*-dimethyl acrylamide, N-ethyl acrylamide, N-propyl acrylamide, N-butyl acrylamide, and *N,N*-diethyl acrylamide; and wherein the acrylate is selected from 2-hydroxymethylacrylate and 2-hydroxyacrylate.

**7.** The method according to any of the preceding claims, wherein the crosslinker component is selected from the group consisting of 2-(2-Diazo-2-phenylacetoxy)ethyl-methacrylate, or 1-(4-(Methacryloyloxy)butyl)-3-methyl-2-diazomalonate; or wherein the crosslinker component is a sulfonyl azide such as 4-styrenesulfonyl azide or 4-propylbenzenesulfonyl azide.

**8.** The method according to any of the preceding claims,
wherein Rc is selected from:

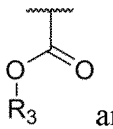
and
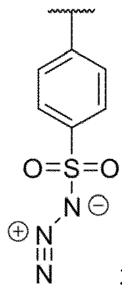
;

wherein $R_3$ is a residue selected from:

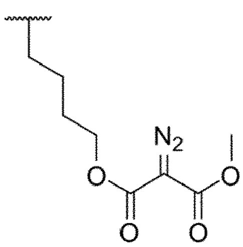
and
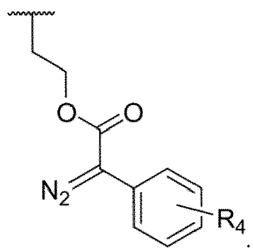
.

wherein $R_4$ is typically selected from H, OH, F, Cl, $CH_3$, $C_2H_5$, and $C_3H_7$.

**9.** The method according to any of the preceding claims, wherein the hydrophilic copolymer comprises at least a content of 2.5 mol% of crosslinker component, typically at least 5 mol% of crosslinker component.

**10.** The method according to any of the preceding claims, wherein step (b) comprises heating the copolymer contacted with the glass fiber to at least 40 °C, specifically to at least 50 °C; more specifically to a temperature between 50 °C and 200 °C.

**11.** The method according to any of the preceding claims, further comprising:

(c) washing the glass fiber fleece; and/or
(d) drying the glass fiber fleece.

**12.** A glass fiber filter, comprising a glass fiber fleece, wherein the surface of the glass fiber fleece is coated with a hydrophilic copolymer.

**13.** A device for biomedical filter applications, typically blood filtration, comprising a glass fiber filter according to any of the preceding claims.

**14.** A method for separating plasma from blood, comprising filtering the blood with a glass fiber filter or device according to any of the preceding claims.

**15.** Use of a hydrophilic copolymer for coating a glass fiber fleece.

**Figure 1**

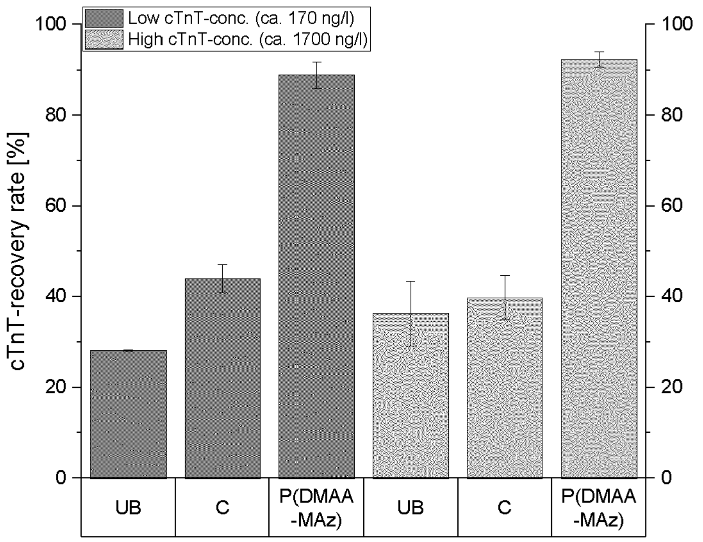


**Figure 2**

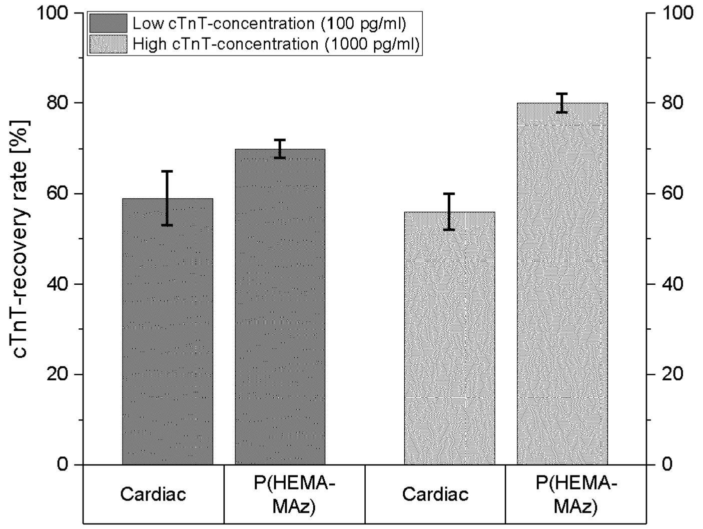

**Figure 3**

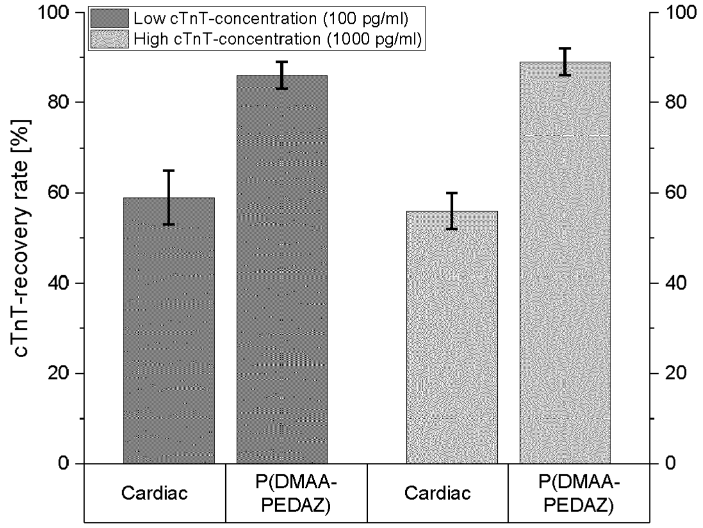


**Figure 4**

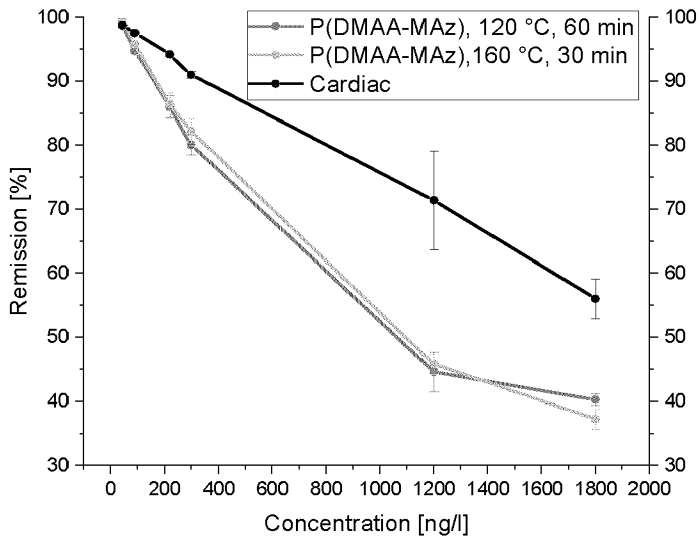

**Figure 5**

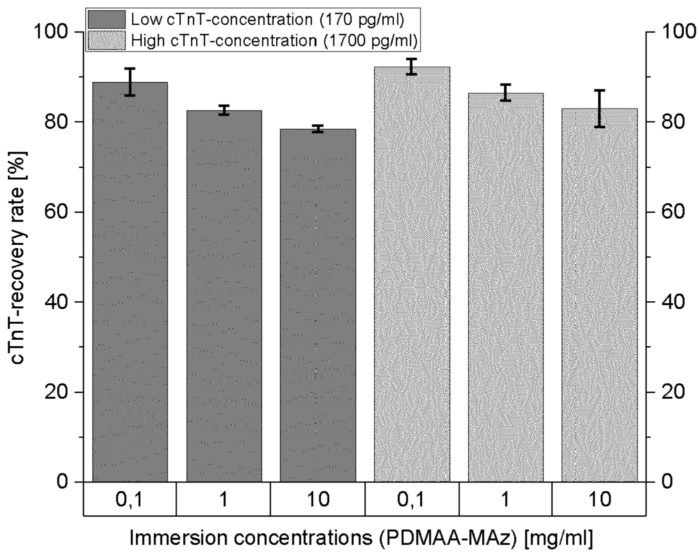


**Figure 6**

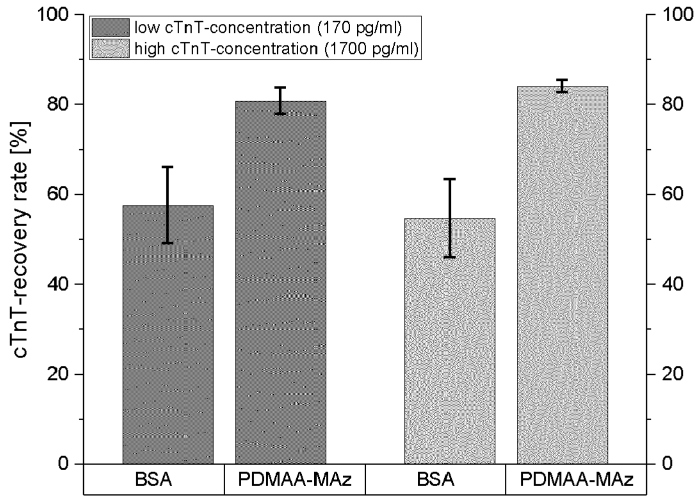

Europäisches Patentamt
European Patent Office
Office européen des brevets

# EUROPEAN SEARCH REPORT

Application Number
EP 23 21 2012

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 102 614 556 A (NANJING RED CROSS BLOOD CT; NANJING SHUANGWEI BIOTECHNOLOGY CO LTD) 1 August 2012 (2012-08-01)<br>* abstract *<br>* paragraphs [0002], [0011], [0012] *<br>* claims 1,2 * | 1,12,13, 15 | INV.<br>B01D67/00<br>B01D39/20<br>B01D69/10<br>B01D71/40<br>B01D71/76<br>G01N33/49<br>G01N33/52 |
| X | US 2016/268566 A1 (DIETZ III ALBERT G [US] ET AL) 15 September 2016 (2016-09-15)<br>* abstract *<br>* claims 1-5,33,42-44 *<br>* paragraphs [0010], [0033], [0057] * | 1,2,12, 13,15 | |
| Y,D | EP 1 618 940 A1 (FUJI PHOTO FILM CO LTD [JP]) 25 January 2006 (2006-01-25)<br>* abstract *<br>* paragraphs [0012], [0013], [0036], [0037] *<br>* claims 1-7 * | 1-15 | |
| Y | WO 2023/069566 A1 (STRECK INC [US]; UNIV FREIBURG ALBERT LUDWIGS [DE]) 27 April 2023 (2023-04-27)<br>* abstract *<br>* paragraphs [0001], [0002], [0004], [0023], [0024], [0035] * | 1-15 | TECHNICAL FIELDS SEARCHED (IPC)<br>B01D<br>G01N |
| A | US 2014/170918 A1 (ANGUS JR RICHARD O [US] ET AL) 19 June 2014 (2014-06-19)<br>* abstract *<br>* claims 4-6, 20,21 * | 1-15 | |

The present search report has been drawn up for all claims

1

| Place of search | Date of completion of the search | Examiner |
|---|---|---|
| Munich | 8 May 2024 | Lançon, Eveline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 21 2012

This annex lists the patent family members relating to the patent documents cited in the above-mentioned European search report. The members are as contained in the European Patent Office EDP file on
The European Patent Office is in no way liable for these particulars which are merely given for the purpose of information.

08-05-2024

| Patent document cited in search report | Publication date | Patent family member(s) | Publication date |
|---|---|---|---|
| CN 102614556 A | 01-08-2012 | NONE | |
| US 2016268566 A1 | 15-09-2016 | CA 2923195 A1 | 09-09-2016 |
| | | EP 3067964 A1 | 14-09-2016 |
| | | ES 2734744 T3 | 11-12-2019 |
| | | PL 3067964 T3 | 31-10-2019 |
| | | SI 3067964 T1 | 30-08-2019 |
| | | TR 201907654 T4 | 21-06-2019 |
| | | US 2016268566 A1 | 15-09-2016 |
| | | US 2017288188 A1 | 05-10-2017 |
| EP 1618940 A1 | 25-01-2006 | EP 1618940 A1 | 25-01-2006 |
| | | JP 2006038512 A | 09-02-2006 |
| | | US 2006016747 A1 | 26-01-2006 |
| WO 2023069566 A1 | 27-04-2023 | NONE | |
| US 2014170918 A1 | 19-06-2014 | CN 105026145 A | 04-11-2015 |
| | | CN 109235052 A | 18-01-2019 |
| | | EP 2931521 A1 | 21-10-2015 |
| | | US 2014170918 A1 | 19-06-2014 |
| | | US 2019263717 A1 | 29-08-2019 |
| | | WO 2014093833 A1 | 19-06-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0457183 A1
- EP 1618940 A1
- JP 2002350428 A


### Non-patent literature cited in the description


- **PRUCKER et al.** *Biointerphases*, January 2018, vol. 13 (1)
- **KOTRADE** ; **RÜHE**. *Angew. Chem. Int. Ed.*, 2017, vol. 56, 14405-14410
- **KOTRADE** ; **RÜHE**. Malonic Acid Diazoesters for C-H Insertion Crosslinking (CHic) Reactions: A Versatile Method for the Generation of Tailor-Made Surfaces. *Angew. Chem. Int. Ed.*, 2017, vol. 56, 14405-14410
- **PRUCKER et al.** Surface-attached hydrogel coatings via C,H-insertion crosslinking for biomedical and bioanalytical applications (Review). *Biointerphases*, January 2018, vol. 13 (1)